# FASCICULE DE BREVET EUROPEEN

(11) **EP 3 744 303 B1**
(45) Date de publication et mention de la délivrance du brevet: **24.01.2024**
(21) Numéro de dépôt: 20176372.9
(22) Date de dépôt: 25.05.2020
(51) Int. Cl.: A61H 23/02, A61L 2/10, A61L 2/24, A46B 17/06

(54) **BASE D ACCUEIL POUR APPAREIL DE SOIN DE LA PEAU AVEC ZONE DE CHARGEMENT ET ZONE DE DESINFECTION**
BASISSTATION FÜR HAUTPFLEGEFERÄT MIT LADE- UND DESINFEKTIONSZONE
DOCKING STATION FOR SKIN CARE DEVICE WITH CHARGING AREA AND DISINFECTION AREA

(30) Priorité: 28.05.2019 FR 1905640
(43) Date de publication de la demande: 02.12.2020
(73) Titulaire: SEB S.A., 69130 Ecully (FR)
(72) Inventeur: CHELLE, Jacky, 38550 AUBERIVES-SUR-VAREZE (FR); MAITRE, Pascal, 07340 FELINES (FR); RENAULT, Fabrice, 69680 CHASSIEU (FR)
(74) Mandataire: SEB Développement

(56) Documents cités:
- WO-A1-2019/045169
- KR-A- 20160 061 666

## Description

### Domaine technique

La présente invention se rapporte au domaine des dispositifs de soin de la peau, et en particulier des appareils conçus pour le nettoyage de la peau et des bases d'accueil pour ces appareils.

Plus particulièrement, l'invention se rapporte au domaine des bases d'accueil pour appareil de soin de la peau, ledit appareil comprenant un corps et un accessoire, le corps comportant un moteur électrique relié à une batterie, le corps pouvant évoluer entre une configuration de travail dans laquelle l'accessoire est accroché sur le corps de sorte à être mis en mouvement par le moteur électrique, et une position de repos dans laquelle l'accessoire est indépendant du corps. La base d'accueil comporte :
- une zone de chargement de l'appareil conçue pour coopérer avec le corps de manière à recharger la batterie,
- une zone de désinfection de l'accessoire conçue pour coopérer avec l'accessoire de manière à désinfecter l'accessoire.

### Technique antérieure

On connait des appareils de soin de la peau, notamment pour le nettoyage de celle-ci, formés d'une part, d'un corps qui comprend une batterie et un moteur électrique, et d'autre part une brosse, accrochée sur le corps et mise en mouvement par le moteur. La forme du corps est telle qu'elle permet à l'utilisateur de manipuler l'appareil de manière à positionner la brosse au contact de la peau afin d'exercer un soin sur cette dernière. Généralement, ce type d'appareil est utilisé pour nettoyer la peau, par exemple afin d'enlever un maquillage, un fond de teint, etc. A cette fin, la brosse est généralement formée de poils qui viennent au contact de la peau. KR 2016 0061666 A concerne un dispositif de charge et de nettoyage d'un appareil de soin de la peau.

Après utilisation de l'appareil, le problème de l'hygiène de l'appareil se pose. En effet, classiquement, la brosse est nettoyée sous l'eau et il est ensuite important que celle-ci puisse sécher dans de bonnes conditions pour limiter le risque de prolifération de bactéries, microbes ou moisissures.

Pour une meilleure facilité d'utilisation, ce genre d'appareil est généralement équipé d'une batterie afin de pouvoir alimenter en électricité le moteur de l'appareil et donc mettre en mouvement la brosse.

Après utilisation de l'appareil, le problème de chargement de la batterie se pose également. En effet, la batterie s'est déchargée pour alimenter le moteur et permettre le soin et il est nécessaire de la recharger.

Afin de résoudre ces différentes problématiques, il a été proposé une base de chargement et de nettoyage décrite dans la demande de brevet WO 2015/168694. Plus précisément, la base de chargement décrite comporte une chambre, c'est-à-dire une cavité, à l'intérieur de laquelle se trouvent d'une part, un dispositif de chargement sans fil (par induction) permettant de recharger la batterie située dans le corps de l'appareil, et, d'autre part, une lampe Ultra-Violet (UV) permettant de désinfecter la brosse fixée au corps de l'appareil.

Ce genre de base donne globalement satisfaction aux utilisateurs notamment grâce à sa simplicité d'utilisation dans la mesure où la brosse et le corps de l'appareil sont disposés dans la chambre de la base. Toutefois, l'hygiène au sein de ce genre de base, et tout particulièrement à l'intérieur de la chambre, peut rester perfectible. En effet, la brosse et une partie du corps de l'appareil se trouvent dans un espace confiné pour ne pas dire clos. Il en résulte des difficultés de séchage de la brosse et du corps de l'appareil, ce qui accroit le risque de prolifération bactérienne, microbienne ou de moisissures sur les zones humides. Cela présentera donc un désagrément voire un risque pour l'utilisateur lors de la prochaine utilisation de l'appareil, d'autant plus si cette prochaine utilisation est éloignée dans le temps (plusieurs jours par exemple) de l'utilisation précédente.

En particulier, la zone située entre le corps de l'appareil et la brosse, c'est-à-dire la zone de liaison entre le corps de l'appareil et la brosse, peut avoir du mal à sécher dans la chambre décrite par ce document. Cette difficulté de séchage est d'autant plus accentuée que la zone située entre le corps de l'appareil et la brosse comporte généralement des renfoncements, des creux, et même des pièces mécaniques complexes (joints, arbre de transmission, etc.) qui peuvent accumuler de l'eau lors du rinçage de l'appareil et qui peuvent ensuite avoir du mal à sécher à l'intérieur de la cavité. En outre, la lumière émise par les lampes UV situées à l'intérieur de la cavité va avoir du mal à atteindre cette zone reculée et ces pièces complexes. Il en résulte un risque de mauvais séchage, de mauvaise désinfection et donc un risque de mauvaise hygiène de cette zone.

En outre, l'hygiène de la chambre décrite dans ce document peut également être perfectible. En effet, en disposant l'accessoire dans cette chambre, il n'est pas exclu que des éléments tels que des peaux mortes, des impuretés ou des résidus de maquillage tombent de la brosse et viennent s'accumuler dans la chambre.

Il existe donc un besoin pour améliorer les dispositifs existants, et en particulier les bases d'accueil des appareils de soin de la peau, notamment au niveau de l'hygiène de ces dernières. La portée de l'invention est définie dans les revendications annexées.

### Exposé de l'invention

La présente invention a pour objectif de pallier les inconvénients précités.

Un objectif de l'invention est de proposer une base d'accueil pour appareil de soin de la peau qui soit particulièrement hygiénique.

Un autre objectif de l'invention est de proposer une base d'accueil pour appareil de soin de la peau qui garantisse un séchage rapide et efficace de l'appareil de soin de la peau.

Un autre objectif de l'invention est de proposer une base d'accueil pour appareil de soin de la peau qui soit particulièrement ergonomique et intuitive à utiliser.

Un autre objectif de l'invention est de proposer une base d'accueil pour appareil de soin de la peau qui soit particulièrement sûre et sans danger.

Un autre objectif de l'invention est de proposer une base d'accueil pour appareil de soin de la peau qui soit particulièrement fiable et robuste.

Un autre objectif de l'invention est de proposer une base d'accueil pour appareil de soin de la peau qui soit particulièrement polyvalente de manière à pouvoir accueillir plusieurs accessoires pour ledit appareil.

Ces objectifs sont atteints à l'aide d'une base d'accueil pour appareil de soin de la peau, ledit appareil comprenant un corps et un accessoire, le corps comportant un moteur électrique relié à une batterie, le corps pouvant évoluer entre une configuration de travail dans laquelle l'accessoire est accroché sur le corps de sorte à être mis en mouvement par le moteur électrique, et une position de repos dans laquelle l'accessoire est indépendant du corps. La base d'accueil comporte alors :
- une zone de chargement de l'appareil conçue pour coopérer avec le corps de manière à recharger la batterie,
- une zone de désinfection de l'accessoire conçue pour coopérer avec l'accessoire de manière à désinfecter l'accessoire.

Selon l'invention, lesdites zone de chargement et zone de désinfection sont distinctes l'une de l'autre.

En outre, préférentiellement, la base d'accueil comprend un dispositif de blocage conçu pour empêcher le corps de coopérer avec la zone de chargement si le corps est en configuration de fonctionnement.

Ainsi, grâce à l'invention, les fonctions de chargement et de désinfection sont dissociées l'une de l'autre grâce à la zone de chargement et à la zone de désinfection distinctes l'une de l'autre. Cela permet d'optimiser chacune de ces fonctions indépendamment l'une de l'autre. En particulier, il est possible de concevoir une zone de désinfection de dimension réduite et parfaitement ajustée aux dimensions de l'accessoire de l'appareil de soin de la peau. Ces dimensions ajustées permettent d'optimiser l'efficacité de la désinfection par rapport à l'art antérieur où la zone de désinfection accueillait également le corps de l'appareil, généralement bien plus volumineux que l'accessoire. En effet, l'espace vide (c'est-à-dire inoccupé) à l'intérieur de la zone de désinfection est minimisé ce qui facilite et optimise la désinfection de l'accessoire, en particulier lorsque la zone de désinfection est équipée de lampe Ultra-Violet et/ou à lumière bleue, comme cela sera détaillé par la suite.

En outre, le dispositif de blocage empêche l'utilisateur de disposer l'appareil de soin de la peau dans la zone de chargement si ce dernier est en configuration de travail. En d'autres termes, si l'appareil de soin de la peau est équipé de l'accessoire (c'est-à-dire si l'accessoire est monté sur le corps de l'appareil), il est impossible pour l'utilisateur de faire coopérer l'appareil de soin de la peau avec la zone de chargement, c'est à dire il est impossible de disposer l'appareil dans la zone de chargement. L'utilisateur est donc obligé de mettre l'appareil en configuration de repos afin de pouvoir placer le corps dans la zone de chargement. Cela évite à l'utilisateur de disposer l'accessoire dans la zone de chargement, évitant ainsi d'une part de contaminer l'accessoire avec d'éventuels impuretés ou microbes présents dans la zone de chargement et d'autre part de contaminer la zone de chargement avec d'éventuels impuretés ou microbes présents sur l'accessoire. Cela permet donc d'améliorer l'hygiène d'une part de la zone de chargement et d'autre part de l'accessoire. En outre, cela incite fortement l'utilisateur (voire même l'oblige) à placer l'accessoire dans la zone de désinfection étant donné que l'utilisateur est obligé d'enlever l'accessoire du corps de l'appareil avant de placer le corps dans la zone de chargement.

La base d'accueil s'étend entre une surface supérieure et une surface inférieure. Par « surface supérieure » on entend la surface qui se situe sur le dessus de la base d'accueil, c'est-à-dire la surface exposée à l'air libre. A l'inverse par « surface inférieure » on entend la surface qui se situe sur le dessous de la base d'accueil, c'est-à-dire la surface qui n'est pas visible, la surface inférieure étant destinée à être posée sur une table ou un plan de travail, généralement par l'intermédiaire de pieds comme cela sera détaillé par la suite.

Préférentiellement, la zone de chargement comporte une chambre s'étendant et ouverte depuis la surface supérieure vers la surface inférieure. En d'autres termes, la chambre est ouverte sur la surface supérieure et s'étend en direction de la surface inférieure, de manière à former une cavité, un creux, à l'intérieur de la base d'accueil, entre lesdites surfaces supérieures et inférieures. Ainsi la chambre permet d'accueillir au moins en partie le corps de l'appareil lorsque ce dernier vient coopérer avec la zone de chargement. En d'autres termes, lorsque le corps de l'appareil coopère avec la zone de chargement pour que cette dernière recharge la batterie, le corps de l'appareil est au moins en partie disposé à l'intérieur de la chambre et donc à l'intérieur de la zone de chargement. Cela permet d'assurer un excellent maintien du corps de l'appareil au sein de la zone de chargement et donc au sein de la base d'accueil, limitant ainsi le risque de chute du corps de l'appareil. Le fait que la chambre soit ouverte sur la surface supérieure de la base d'accueil permet d'améliorer l'ergonomie et la facilité d'utilisation de la base d'accueil puisque l'utilisateur n'a aucune manipulation à faire : il peut directement disposer le corps de l'appareil dans la zone de chargement.

Selon cette réalisation préférentielle, le dispositif de blocage comporte une surface pleine située en retrait de la surface supérieure de manière à fermer la chambre en direction de la surface inférieure. Cette surface pleine permet alors de remplir une double fonction : d'une part elle oblige l'utilisateur à démonter l'accessoire du corps de l'appareil (c'est-à-dire à mettre l'appareil de soin de la peau dans sa configuration de repos) avant de faire coopérer le corps de l'appareil avec la zone de chargement, et d'autre part elle permet de supporter le poids du corps de l'appareil lorsque ce dernier coopère avec la zone de chargement. En effet, le corps de l'appareil peut alors reposer sur une surface plane et pleine ce qui permet de bien répartir le poids du corps et ainsi améliorer la stabilité et le maintien du corps de l'appareil lorsque ce dernier coopère avec la zone de chargement.

Avantageusement, la zone de chargement comporte un dispositif de chargement sans contact, préférentiellement par induction. Ledit dispositif de chargement sans contact va alors coopérer avec un dispositif de réception complémentaire, lui aussi sans contact situé à l'intérieur du corps de l'appareil. Cela permet de transférer l'énergie électrique sans contact, de manière particulièrement sûre et hygiénique. En effet, la zone de chargement est ainsi dépourvue de contacts métalliques pour recharger la batterie du corps de l'appareil ce qui élimine tout risque d'oxydation, de rouille desdits contacts.

Préférentiellement, la zone de désinfection comporte une cavité s'étendant et ouverte depuis la surface supérieure vers la surface inférieure. En d'autres termes, la cavité est ouverte sur la surface supérieure et s'étend en direction de la surface inférieure, de manière à former une chambre, un creux, à l'intérieur de la base d'accueil, entre lesdites surfaces supérieures et inférieures. Ainsi, la cavité permet d'accueillir au moins en partie l'accessoire de l'appareil lorsque ce dernier vient coopérer avec la zone de désinfection. En d'autres termes, lorsque l'accessoire de l'appareil coopère avec la zone de désinfection pour que cette dernière désinfecte l'accessoire, l'accessoire de l'appareil est au moins en partie disposé à l'intérieur de la cavité et donc à l'intérieur de la zone de désinfection. Cela permet d'assurer un excellent maintien de l'accessoire de l'appareil au sein de la zone de désinfection et donc au sein de la base d'accueil, limitant ainsi le risque de chute de l'accessoire. Cela permet également d'améliorer l'efficacité de la désinfection de l'accessoire dans la mesure où celle-ci s'effectue dans un espace confiné. Le fait que la cavité soit ouverte sur la surface supérieure de la base d'accueil permet d'améliorer l'ergonomie et la facilité d'utilisation de la base d'accueil puisque l'utilisateur n'a aucune manipulation à faire : il peut directement disposer l'accessoire de l'appareil dans la zone de désinfection.

Avantageusement, selon cette réalisation préférentielle, la cavité est également ouverte du côté de la surface inférieure. En d'autres termes, contrairement à la zone de chargement, la zone de désinfection comporte au moins une ouverture du côté de la surface inférieure de la base d'accueil. Cela permet de faire circuler l'air à l'intérieur de la zone de désinfection et donc d'améliorer le séchage de l'accessoire, et donc l'hygiène de ce dernier. Cela est particulièrement appréciable et nécessaire lorsque l'accessoire est formé d'une brosse qui comporte une pluralité de poils.

Toujours selon la réalisation préférentielle précédente, de manière avantageuse, la zone de désinfection comporte un dispositif d'arrêt conçu pour coopérer avec l'accessoire de manière à supporter ce dernier au sein de la cavité. Avantageusement, le dispositif d'arrêt comporte un chanfrein ce qui permet de bien répartir le poids de l'accessoire dans la zone de désinfection et donc d'améliorer le maintien de l'accessoire. De manière alternative ou préférentiellement complémentaire, le dispositif d'arrêt comporte des ergots d'appui ce qui permet d'éviter une étanchéité entre l'accessoire et le chanfrein. Cela permet ainsi de faire circuler l'air, voire même de créer une sorte de cheminée, ce qui optimise le séchage de l'accessoire à l'intérieur de la cavité.

De manière avantageuse, la zone de désinfection comporte une lampe, éventuellement une lampe UV, émettant une lumière bleue. En effet, il est connu que la lumière avec une longueur d'onde dans la couleur bleue et/ou les UV (rayons Ultra-Violet) ont des effets désinfectants notoires. Préférentiellement, la zone de désinfection comporte un indicateur visuel conçu pour permettre à un utilisateur de vérifier l'allumage de la lampe. Cela permet d'informer l'utilisateur sur la désinfection de son accessoire ou au contraire de l'alerter d'une non désinfection si la lampe n'est pas allumée. De manière particulièrement avantageuse, l'indicateur visuel est formé par le chanfrein.

Préférentiellement, la zone de désinfection comporte un dispositif de commande spécifique conçu pour contrôler l'allumage et l'extinction de ladite lampe émettant une lumière bleue de manière automatique et indépendamment du contrôle de la zone de chargement. Ainsi, il est remarquablement possible d'alimenter la zone de désinfection, c'est-à-dire d'allumer ladite lampe émettant une lumière bleue pour procéder au nettoyage de l'accessoire, même si la zone de chargement n'est pas alimentée. Cela permet de nettoyer l'accessoire même s'il n'est pas nécessaire de recharger l'appareil.

Préférentiellement, ledit dispositif de commande spécifique comprend un compteur de temps conçu pour être déclenché lorsque la zone de chargement et le corps coopèrent et pour envoyer un signal au dispositif de commande spécifique lorsqu'une durée seuil est atteinte. Ladite durée seuil est préférentiellement fixée à 3h, cette durée ayant été calculée pour garantir un nettoyage optimal de l'accessoire. Le dispositif de commande spécifique est en outre conçu pour éteindre la lampe émettant une lumière bleue lorsque la durée seuil est atteinte.

Selon un premier mode de réalisation avantageux, la base d'accueil consiste en une zone de chargement et une zone de désinfection, lesdites zone de chargement et de désinfection étant distinctes l'une de l'autre, c'est-à-dire séparées l'une de l'autre. En d'autres termes, selon ce premier mode de réalisation, la base d'accueil comporte exactement deux zones distinctes : une zone de chargement destinée à coopérer avec le corps de l'appareil et une zone de chargement destinée à coopérer avec l'accessoire de l'appareil.

Selon un deuxième mode de réalisation de l'invention, la base d'accueil comprend en outre une zone de stockage conçue pour coopérer avec l'accessoire ou un autre accessoire de manière à assurer le maintien de ce dernier. Cela permet de proposer une base d'accueil capable d'accueillir simultanément deux accessoires et le corps de l'appareil. Préférentiellement, selon ce mode de réalisation, la base d'accueil consiste en trois zones : la zone de chargement, la zone de désinfection et une zone de stockage. Avantageusement ces trois zones sont distinctes chacune les unes des autres, ce qui permet de bien distinguer et optimiser chacune des fonctions de chargement, de désinfection et de stockage, comme cela a été expliqué précédemment.

Préférentiellement, ledit accessoire ou un autre accessoire comporte une brosse, c'est-à-dire une zone comportant une pluralité de poils ou picots plus ou moins rigides destinés à venir au contact de la peau. Différents matériaux, différentes formes, ou encore différentes longueurs peuvent être utilisés pour former les poils ou les picots ce qui permet de proposer différents soins pour la peau de l'utilisateur.

Selon ce deuxième mode de réalisation, avantageusement, la zone de stockage comporte un plot conçu pour coopérer avec ledit accessoire ou un autre accessoire de manière à ce que la brosse soit stockée à l'air libre. Cela permet de garantir un séchage parfait de la brosse de l'accessoire et de limiter ainsi tout risque de prolifération bactérienne. En d'autres termes, le plot agit comme un obstacle qui empêche l'utilisateur de disposer la brosse dans la zone de stockage.

Préférentiellement, ledit accessoire ou un autre accessoire comporte un moyen d'accrochage, ce dernier étant destiné à correspondre avec un moyen d'accrochage complémentaire embarqué sur le corps de l'appareil de soin de la peau. Dans ce cas, de manière remarquable, le plot est conçu pour accueillir autour de lui le dispositif d'accrochage.

Toujours selon ce deuxième mode de réalisation, selon un arrangement avantageux, lesdites zone de chargement, zone de désinfection et zone de stockage sont disposées de manière à former un triangle. Cela permet de remarquablement optimiser l'espace au sein de la base d'accueil tout en minimisant l'encombrement de cette dernière. En outre, l'ergonomie et l'intuitivité d'utilisation sont améliorées puisque chaque zone est située à proximité d'un sommet du triangle.

Selon cet arrangement avantageux, préférentiellement, ledit triangle est un triangle isocèle dont la base est formée par lesdites zone de désinfection et zone de stockage et dont le sommet est formé par ladite zone de chargement. Compte tenu de la taille sensiblement identique des zone de stockage et zone de désinfection (elles sont toutes les deux conçues pour coopérer avec l'accessoire ou un autre accessoire), cela permet de donner une symétrie à la base d'accueil.

### Brève description des dessins

La figure 1 est une vue d'ensemble en perspective d'une base d'accueil selon un premier mode de réalisation de l'invention.
La figure 2 représente la base d'accueil de la figure 1 dans laquelle est disposé un appareil de soin de la peau.
La figure 3 est une vue en perspective éclatée de la figure 2.
La figure 4 est une vue de dessus de la base d'accueil de la figure 1.
La figure 5 est une vue de dessous de la base d'accueil de la figure 1.
La figure 6 est une vue en coupe et en perspective de la base d'accueil de la figure 1.
La figure 7 est une vue en perspective d'un appareil de soin de la peau que peut accueillir la base d'accueil telle que définie par l'invention. Cette figure illustre le corps de l'appareil de soin de la peau en position de travail.
La figure 8 est une vue en perspective de deux éléments composant l'appareil de soin de la peau de la figure 7 : un corps et un accessoire. Cette figure illustre le corps de l'appareil de soin de la peau en position de repos.
La figure 9 est une vue en perspective éclatée montrant une base d'accueil définie selon un deuxième mode de réalisation de l'invention et un appareil de soin de la peau comprenant un corps et deux accessoires.
La figure 10 est une vue de dessus de la base d'accueil de la figure 9.
La figure 11 est une vue de dessous de la base d'accueil de la figure 9.

### Description des modes de réalisation

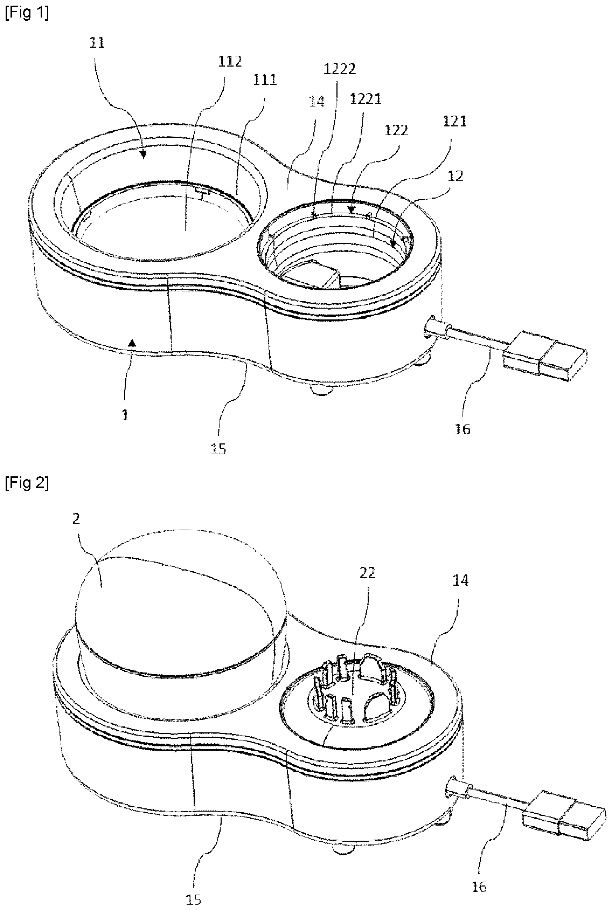

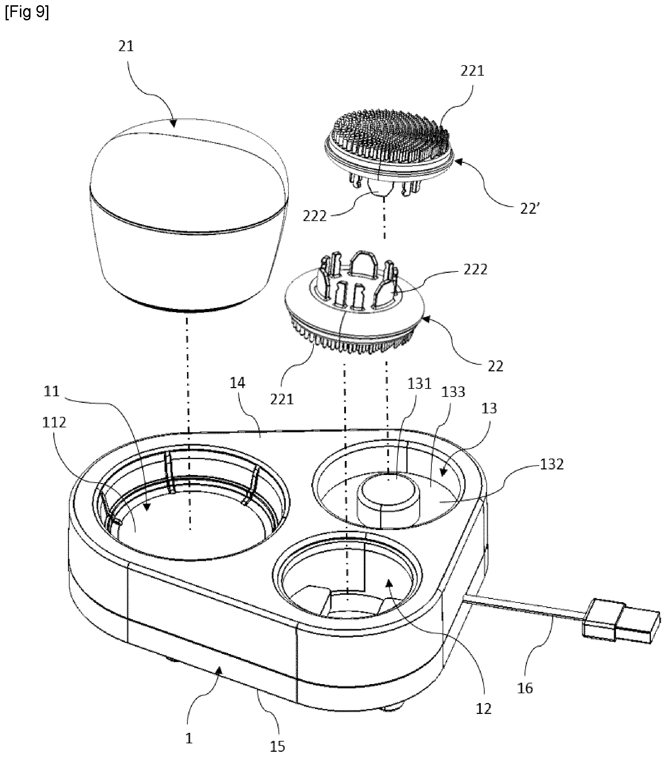

Un premier mode de réalisation de l'invention est illustré par les figures 1 à 6. Un deuxième mode de réalisation est illustré par les figures 9 à 11. Lesdites figures montrent une base d'accueil 1 pour appareil 2 préférentiellement de soin de la peau.

La base d'accueil 1 et l'appareil 2 de soin de la peau forment ensemble un dispositif de soin de la peau, ladite base d'accueil 1 étant conçue pour à la fois recevoir, désinfecter, charger et éventuellement stocker l'appareil 2 de soin de la peau. En d'autres termes, l'appareil 2 de soin de la peau est indépendant et distinct de la base d'accueil 1 bien que ces deux éléments soient conçus pour coopérer l'un avec l'autre, comme cela sera détaillé par la suite.

Selon le premier mode de réalisation de l'invention, la base d'accueil 1 comporte précisément deux zones distinctes, c'est-à-dire deux zones différentes et distantes l'une de l'autre. Comme on peut le voir sur les figures 1 à 6, chacune de ces zones est de forme circulaire et défini un évidement, c'est-à-dire un creux, comme cela sera détaillé par la suite. La base d'accueil 1 comporte ainsi une zone de chargement 11 qui sera détaillée ci-après et une zone de désinfection 12 qui sera également décrite par la suite.

Selon un deuxième mode de réalisation de l'invention, la base d'accueil 1 comporte précisément trois zones distinctes, dont une zone de stockage 13, une zone de désinfection 12 et une zone de chargement 11 comme cela sera détaillé par la suite. Dans ce cas, la base d'accueil 1 peut prendre la forme d'un triangle comme illustré aux figures 9 à 11, et plus précisément la forme d'un triangle isocèle. En effet, lesdites zone de chargement 11, zone de désinfection 12 et zone de stockage 13 sont disposées de manière à former un triangle. Plus précisément, ledit triangle est un triangle isocèle dont la base est formée par lesdites zone de désinfection 12 et zone de stockage 13 et dont le sommet est formé par ladite zone de chargement 11. En effet, cette forme est particulièrement bien adaptée dans la mesure où les zone de désinfection 12 et de stockage 13 ont un diamètre similaire (en effet elles sont toutes les deux conçues pour accueillir un accessoire 22). La zone de chargement 11 a un diamètre plus important.

Quel que soit le mode de réalisation, la base d'accueil 1 s'étend entre une surface supérieure 14 et une surface inférieure 15. La surface inférieure 15 est dans ce cas définie comme étant la surface disposée au contact ou au moins proche d'un plan d'appui, comme une table par exemple. La surface supérieure 14 est par opposé la surface la plus éloignée du plan d'appui (de la table par exemple) et est la surface la plus visible, la surface la plus exposée à l'air libre.

Comme on peut le voir sur les différentes figures, lesdites surfaces supérieure 14 et inférieure 15 sont des surfaces sensiblement planes et parallèles entre elles.

L'épaisseur de la base d'accueil 1, c'est-à-dire la distance entre la surface supérieure 14 et la surface inférieure 15 est avantageusement comprise entre 15 mm et 50 mm, préférentiellement comprise entre 25 mm et 40 mm, par exemple en étant sensiblement égale à 32.8 mm.

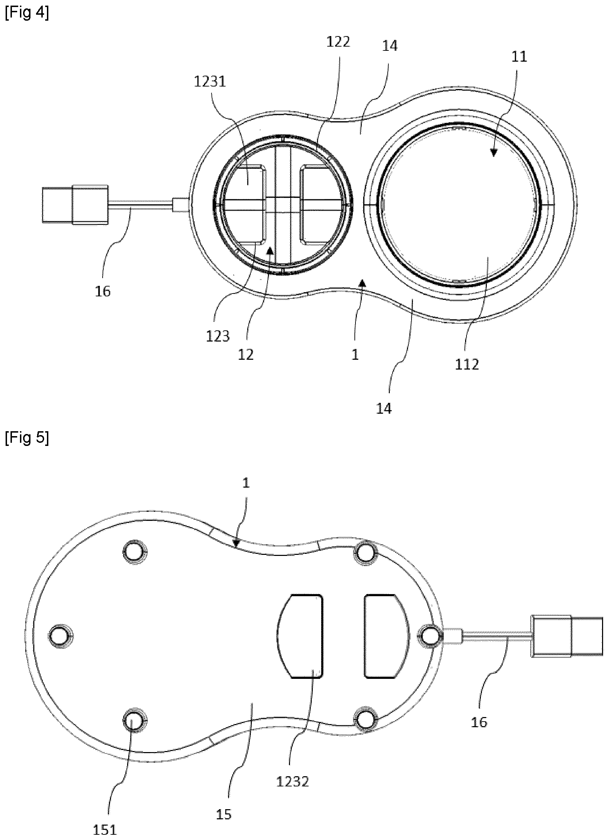

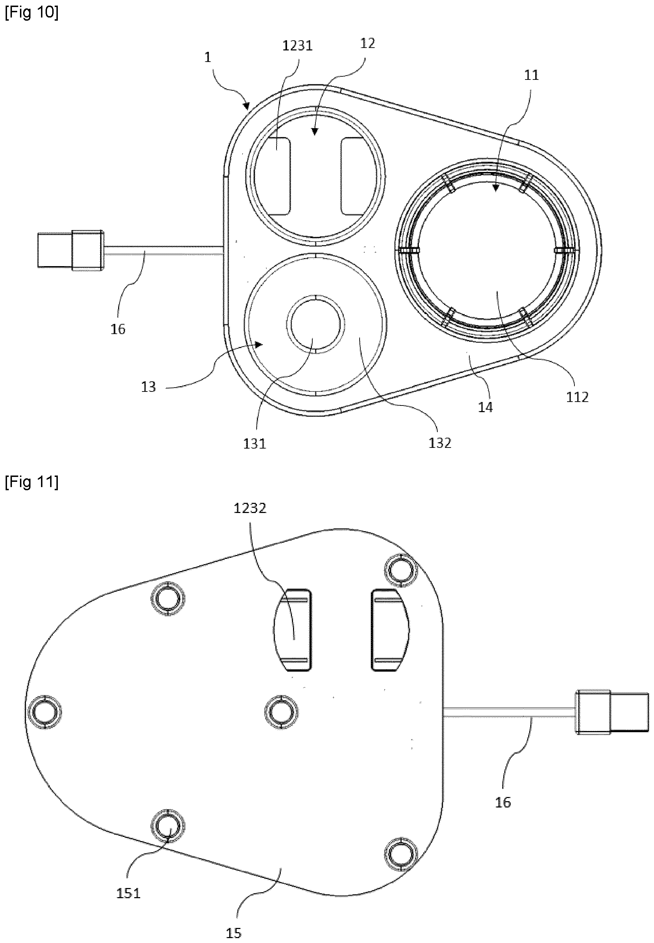

La surface inférieure 15 peut comporter des pieds 151 comme on peut le voir sur la figure 5 ou la figure 11 de manière à décoller la surface inférieure 15 du plan d'appui, généralement une table ou un plan de travail.

La surface supérieure 14 comporte au moins deux ouvertures formant lesdites zone de chargement 11 et zone de désinfection 12 comme cela sera détaillé par la suite. Ainsi, lesdites zone de chargement 11 et zone de désinfection 12 s'étendent sous forme de chambres ou de cavités à l'intérieur de la base d'accueil 1, entre lesdites surface supérieure 14 et inférieure 15 comme cela sera décrit par la suite.

Dans le cas particulier du deuxième mode de réalisation, la surface supérieure 14 comporte trois ouvertures formant lesdites zone de chargement 11, zone de désinfection 12 et zone de stockage 13, comme cela sera détaillé par la suite. Ainsi, lesdites zone de chargement 11, zone de désinfection 12 et zone de stockage 13 s'étendent sous forme de chambres ou de cavités à l'intérieur de la base d'accueil 1, entre lesdites surface supérieure 14 et inférieure 15 comme cela sera décrit par la suite.

La zone de chargement 11 et la zone de désinfection 12 sont chacune de forme circulaire mais sont de diamètres différents. Plus précisément, le diamètre de la zone de chargement 11 est supérieur au diamètre de la zone de désinfection 12 ce qui confère à la base d'accueil 1 une forme de cacahuète (ou arachide) comme on peut le voir sur la figure 4 par exemple.

Dans le cas particulier du deuxième mode de réalisation, la zone de stockage 13 est également de forme circulaire et son diamètre est sensiblement égal à celui de la zone de désinfection 12.

D'après les figures 4 et 5 et le premier mode de réalisation, il est possible de définir une longueur et une largeur à la base d'accueil 1. La longueur de la base d'accueil 1 est par exemple comprise entre 100 mm et 160 mm en étant par exemple sensiblement égale à 132.4 mm. La largeur de la base d'accueil 1 peut être comprise entre 60 mm et 100 mm et sensiblement égale à 80.6 mm au niveau de la zone de chargement 11 ou entre 40 mm et 80 mm et sensiblement égale à 64 mm au niveau de la zone de désinfection 12.

On pourrait bien entendu imaginer, sans sortir du cadre de l'invention, d'autres bases d'accueil qui comporteraient plus que deux (premier mode de réalisation) ou trois (deuxième mode de réalisation) zones distinctes. Par exemple on pourrait imaginer une base d'accueil 1 comportant quatre zones distinctes, cinq zones distinctes, etc.

Comme on peut le voir sur les différentes figures, et quel que soit le mode de réalisation, la base d'accueil 1 comporte également un moyen d'alimentation électrique 16 comportant lui-même un câble électrique et un connecteur, par exemple tel que défini par la norme USB.

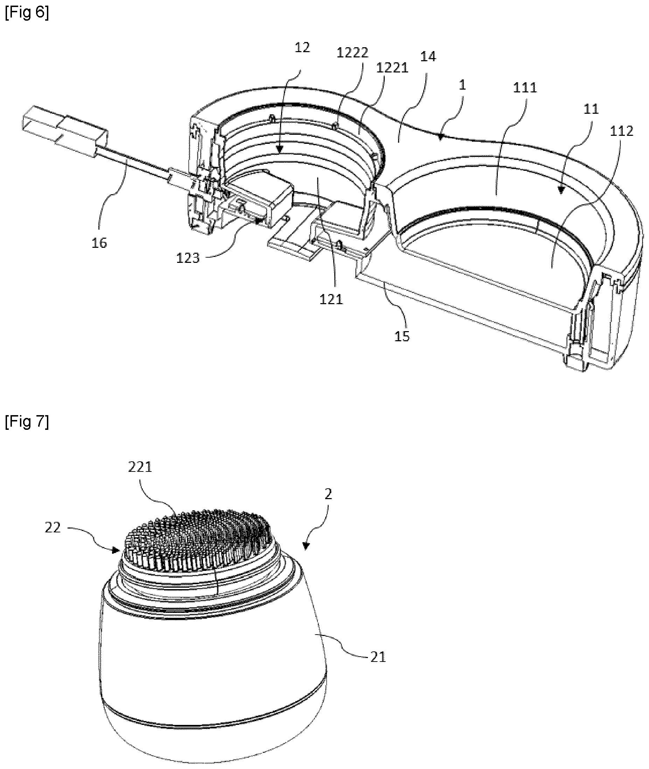

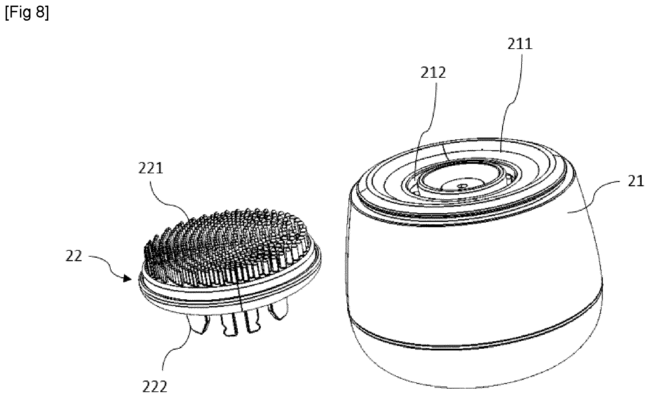

Un appareil 2 de soin de la peau particulièrement adapté pour correspondre avec ladite base d'accueil 1 est illustré aux figures 7 et 8. Plus précisément, comme on peut le voir sur ces figures, l'appareil 2 comprend un corps 21 et un accessoire 22.

Préférentiellement, comme le montre la figure 8, l'accessoire 22 est amovible du corps 21, c'est-à-dire qu'il peut être, selon la volonté de l'utilisateur de l'appareil 2 de soin de la peau, être monté ou démonté (c'est-à-dire assemblé ou désassemblé) du corps 21 de l'appareil.

De manière connue en tant que telle, l'accessoire 22 comporte une brosse 221. Cette dernière est généralement formée d'une pluralité de poils plus ou moins souples destinés à venir au contact de la peau de l'utilisateur afin d'appliquer un traitement sur cette dernière. Les poils peuvent être réalisés en matériau synthétique et/ou naturels. Généralement la brosse 221 est utilisée pour effectuer un nettoyage de la peau de l'utilisateur, par exemple pour enlever de cette dernière un maquillage, un fond de teint, etc. Eventuellement, en fonction de la dureté des poils de la brosse 221, la brosse 221 peut être utilisée pour effectuer diverses opérations de soins esthétiques comme par exemple un gommage, une exfoliation, etc. De manière alternative ou complémentaire aux poils, la brosse 221 peut également comprendre des picots, par exemple en silicone, qui permettent de proposer un accessoire avec d'autres propriétés mécaniques et donc un autre soin pour la peau de l'utilisateur. Toutefois, on pourrait imaginer sans sortir du cadre de l'invention, un accessoire 22 qui ne serait pas équipé d'une brosse 221 mais d'un autre dispositif adapté pour effectuer une opération de soin de la peau, en particulier un massage. Par exemple, de manière illustrative et non limitative, l'accessoire 22 peut comporter une mousse, une ou plusieurs billes, des rouleaux, des doigts, une combinaison de picots et d'orifices de distribution d'un produit, etc.

L'accessoire 22 comporte un moyen d'accrochage 222 destiné à correspondre avec un moyen d'accrochage complémentaire 212 situé sur le corps 21. Plus précisément, comme on peut le voir sur la figure 8, le moyen d'accrochage 222 est un moyen d'accrochage mâle et comporte une pluralité d'ergots disposés de manière circulaire, tandis que le moyen d'accrochage complémentaire 212 est un moyen d'accrochage femelle qui comporte une rainure circulaire. Ces moyens d'accrochage 222 et moyens d'accrochage complémentaires 212 sont bien adaptés pour transmettre un mouvement de rotation et/ou d'oscillation et sont bien connus en tant que tels si bien qu'ils ne seront pas d'avantage décrits ici. On pourrait toutefois imaginer, sans sortir du cadre de l'invention, équiper l'accessoire 22 et le corps 21 d'autres couples de moyens d'accrochages complémentaires entre eux.

Ainsi, le corps 21 peut se trouver dans une configuration de travail dans laquelle l'accessoire 22 est accroché sur le corps 21, comme on peut le voir sur la figure 7. Dans cette configuration de travail, un utilisateur peut saisir le corps 21 de l'appareil 2 dans sa main pour approcher l'appareil 2 de sa peau, en particulier de son visage. L'utilisateur va alors disposer l'accessoire 22 de l'appareil 2 au contact de sa peau, par exemple au contact de la peau de son visage. Il convient de noter que la forme du corps 21 de l'appareil 2 est telle qu'elle permet une saisie aisée et sûre par la main de l'utilisateur, par exemple en permettant de placer le corps 21 au centre de la paume de la main comme on peut le comprendre à partir des figures 7 et 8.

De manière connue en tant que telle, le corps 21 comporte un moteur électrique relié à une batterie. Lorsque l'accessoire 22 est monté sur le corps 21, c'est-à-dire lorsque l'appareil 2 est dans sa configuration de travail, l'accessoire 22 peut être mis en mouvement par le moteur électrique, par exemple un mouvement de rotation ou encore d'oscillation. Le moteur électrique est lui-même alimenté en énergie par la batterie. Le terme « batterie » englobe n'importe quel dispositif de stockage ou de génération d'électricité, comme par exemple un dispositif de génération d'électricité par réaction chimique (comme c'est par exemple le cas dans une batterie ou une pile), mais également d'autres dispositifs tels que, de manière illustrative et non limitative une capacité, une super capacité, etc. Ces éléments et ces mouvements sont bien connus en tant que tels et ne seront donc pas d'avantage détaillés ici.

Le corps 21 peut aussi se trouver dans une position de repos dans laquelle l'accessoire 22 est indépendant du corps 21, comme on peut le voir sur la figure 8. Dans cette position de repos, l'accessoire 22 n'est pas monté sur le corps 21 si bien que l'accessoire 22 ne peut pas être mis en mouvement par le moteur. Le corps 21 est alors dans une position optimale permettant de recharger sa batterie. En effet, le corps 21 comporte une zone d'échange 211 située sur la face supérieure du corps 21 accueillant l'accessoire 22 en position de travail. Ainsi, la zone d'échange 211 n'est accessible que lorsque le corps 21 est en position de repos. En effet, lorsque le corps 21 est en position de travail, la zone d'échange 211 est recouverte par l'accessoire 22 comme le montrent les figures 7 et 8.

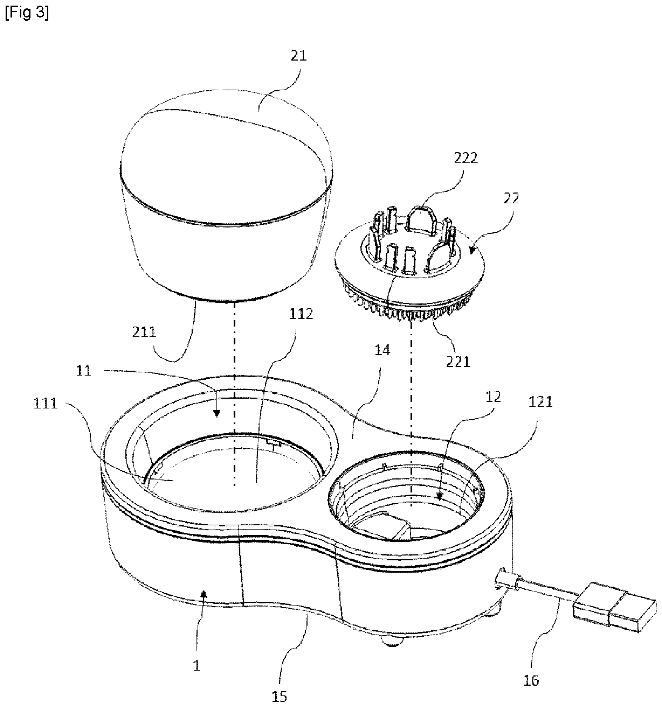

Comme illustré, la base d'accueil 1 comporte une zone de chargement 11 de l'appareil 2 conçue pour coopérer avec le corps 21 de manière à recharger la batterie. En d'autres termes, lorsque l'utilisateur veut recharger la batterie de son appareil 2 de soin de la peau, il doit placer ce dernier dans la zone de chargement 11 de la base d'accueil 1. Plus précisément, comme le montrent les figures 2 et 3, le corps 21 est disposé de sorte à faire coïncider la zone d'échange 211 avec la zone de chargement 11.

Comme on peut le voir sur les figures 1, 3 et 6 notamment, la zone de chargement 11 comporte une chambre 111, c'est-à-dire un espace creux, une cavité. Cette chambre 111 s'étend depuis la surface supérieure 14 vers la surface inférieure 15. En outre, cette chambre 111 est ouverte depuis la surface supérieure 14. En d'autres termes, la zone de chargement 11 est formée par une chambre 111 s'étendant à l'intérieur de la base d'accueil 1, depuis la surface supérieure 14 en direction de la surface inférieure 15. La chambre 111 est ouverte, c'est-à-dire débouchante, sur la surface supérieure 14. A l'inverse, la zone de chargement 11 est fermée, c'est-à-dire non débouchante, sur la surface inférieure 15 comme on peut le voir sur les figures 1, 3 et 6 notamment. Plus précisément, la chambre 111 est fermée par une surface pleine. En d'autres termes, la chambre 111 forme un trou non débouchant. Cette surface pleine constitue alors un dispositif de blocage 112 comme on peut le voir sur différentes figures.

La zone de chargement 11 comprend donc un dispositif de blocage 112 qui permet de retenir le corps 21 de l'appareil 2 de soin de la peau. Ainsi, la base d'accueil 1 comprend un dispositif de blocage 112. Ce dernier est, comme expliqué, conçu pour supporter le corps 21 de l'appareil 2 de soin de la peau. Le dispositif de blocage 112 est en outre conçu pour empêcher le corps 21 de coopérer avec la zone de chargement 11 si le corps 21 est en configuration de fonctionnement.

En effet, si le corps 21 est dans sa position de repos, c'est-à-dire si le corps 21 et l'accessoire 22 sont assemblés comme sur la figure 7, alors il est impossible pour l'utilisateur de procéder au rechargement de la batterie du corps 21. En effet, compte tenu de la présence de l'accessoire 22 sur le corps 21, la zone d'échange 211 est cachée et ne peut pas coopérer avec la zone de chargement 11. Par conséquent la recharge de la batterie ne peut pas avoir lieu.

Ainsi, la base d'accueil 1, grâce à son dispositif de blocage 112 va empêcher l'utilisateur de positionner l'appareil 2 de soin de la peau dans la zone de chargement 11 dans sa configuration de travail. Ainsi, de manière tout à fait remarquable, l'hygiène de l'appareil 2 de soin de la peau, et en particulier de l'accessoire 22, est grandement améliorée puisque chacun desdits corps 21 et accessoire 22 vont pouvoir sécher de manière optimale et indépendamment les uns des autres. En particulier, en obligeant l'utilisateur à mettre l'appareil 2 de soin de la peau dans sa configuration de repos avant de le positionner à l'intérieur de la zone de chargement 11, la base d'accueil 1 permet, contrairement aux dispositifs connus de l'art antérieur, d'éviter l'accumulation d'eau de rinçage ou de résidus (de peau morte, de maquillage, etc.) dans des creux ou renfoncements, en particulier ceux situés entre le corps 21 et l'accessoire 22. Cela améliore donc la faculté de séchage de l'appareil 2 de soin de la peau du corps 21 et de l'accessoire 22 et donc l'hygiène globale de l'appareil 2 de soin de la peau.

Comme expliqué, le dispositif de blocage 112 comporte une surface pleine. Comme on peut le voir sur les figures, le dispositif de blocage 112 peut être formé par une surface plane, comme un disque par exemple. Cela permet de bien répartir le poids du corps 21 lorsque ce dernier se trouve dans sa position de repos ce qui contribue à améliorer la stabilité du corps 21 lorsque ce dernier se trouve dans la zone de chargement 11.

En outre, comme on peut le voir sur les figures 1, 3, 6, le dispositif de blocage 112, en l'occurrence la surface pleine, est située en retrait de la surface supérieure 14 de manière à fermer la chambre 111 en direction de la surface inférieure 15. En d'autres termes, le dispositif de blocage 112 est décalé par rapport à la surface supérieure 14. Le dispositif de blocage 112 est donc situé entre la surface supérieure 14 et la surface inférieure 15, par exemple en étant à mi-chemin entre lesdites surfaces.

Ainsi, comme expliqué précédemment, la zone de chargement 11 forme une chambre 111 ouverte sur la surface supérieure 14 mais fermée du côté de la surface inférieure 15. Cela permet de disposer une partie au moins du corps 21 à l'intérieur de la chambre 111. Ainsi le corps 21 est bien maintenu et le risque de chute de ce dernier lorsqu'il se trouve dans la zone de chargement 11 est remarquablement réduit ce qui améliore la sécurité de la base d'accueil 1, y compris si l'utilisateur venait à déplacer celle-ci alors que le corps 21 était dans la zone de chargement 11.

Avantageusement, la zone de chargement 11 comporte un dispositif de chargement sans contact, préférentiellement par induction. Cela permet d'éviter la présence de contacteurs métalliques entre la zone de chargement 11 et le corps 21 et donc le risque de corrosion de ces derniers. L'hygiène et la fiabilité de la base d'accueil 1 et de l'appareil 2 de soin de la peau est donc améliorée. Le dispositif de chargement sans contact peut par exemple être située sous le dispositif de blocage 112, entre ce dernier et la surface inférieure 15. Ainsi, lorsque le corps 21 est disposé dans la zone de chargement 11, le dispositif de chargement sans contact, par exemple une bobine d'induction, se trouve en dessous et en face de la zone d'échange 211 ce qui améliore la fiabilité de la recharge.

La base d'accueil 1 comporte également une zone de désinfection 12, ou zone de nettoyage, de l'accessoire 22. L'objectif de cette dernière est de contribuer à désinfecter, à nettoyer l'accessoire 22, par exemple en détruisant au moins une partie des microbes, bactéries ou autres agents pathogènes qui pourraient se trouver sur l'accessoire 22, et en particulier sur la brosse 221, comme expliqué précédemment. La zone de désinfection 12 est donc conçue pour coopérer avec l'accessoire 22 de manière à désinfecter l'accessoire 22 même si l'on pourra admettre que la désinfection n'est pas totale et ne peut atteindre les niveaux requis dans le domaine médical.

Comme on peut le voir sur les figures 1, 3 et 6, la zone de désinfection 12 comporte une cavité 121 c'est-à-dire un espace creux, une chambre. Cette cavité 121 s'étend depuis la surface supérieure 14 vers la surface inférieure 15. En outre, cette cavité 121 est ouverte depuis la surface supérieure 14. En d'autres termes, la zone de désinfection 12 est formée par une cavité 121 s'étendant à l'intérieur de la base d'accueil 1, depuis la surface supérieure 14 en direction de la surface inférieure 15. La cavité 121 est ouverte, c'est-à-dire débouchante, sur la surface supérieure 14.

Contrairement à la zone de chargement 11, la cavité 121 est ouverte du côté de la surface inférieure 15. En d'autres termes, la zone de désinfection 12 est une zone débouchante du côté de la surface inférieure 15 tout en étant également ouverte sur la surface supérieure 14. Cela permet d'avoir une excellente circulation d'air à l'intérieur de la cavité 121 et donc au sein de la zone de désinfection 12. Cela garantit un excellent séchage de l'accessoire 22 qui est placé dans la zone de désinfection 12.

Plus précisément, comme on peut le voir sur la figure 6, la zone de désinfection 12 comporte un dispositif d'arrêt 122 conçu pour coopérer avec l'accessoire 22 de manière à supporter ce dernier au sein de la cavité 121. En d'autres termes, le dispositif d'arrêt 122 permet de supporter le poids de l'accessoire 22 et donc d'empêcher ce dernier de passer au travers de la cavité 121. Plus précisément, de manière préférentielle, le dispositif d'arrêt 122 comporte un chanfrein 1221 comme on peut le voir par exemple sur la figure 6. Cela permet de bien guider l'accessoire 22 au sein de la cavité 121 et donc au sein de la zone de désinfection 12 lorsque l'utilisateur pose l'accessoire 22 sur la base d'accueil 1. Ainsi l'ergonomie d'utilisation de la base d'accueil 1 est améliorée. En outre, le dispositif d'arrêt 122 permet également d'assurer un très bon maintien de l'accessoire 22 dans la zone de désinfection 12, y compris en cas de déplacement de la base d'accueil 1.

Préférentiellement, le dispositif d'arrêt 122 comporte également des ergots d'appui 1222 visibles notamment sur la figure 6. Ces derniers peuvent faire saillie du chanfrein 1221. Ces ergots d'appui 1222 permettent de décoller légèrement l'accessoire 22 du dispositif d'arrêt 122 et donc de garantir une circulation d'air au sein de la zone de désinfection 12, à la manière d'une cheminée. Cela améliore encore le séchage de l'accessoire 22 et donc l'hygiène de ce dernier.

Comme on peut le voir sur la figure 6, la cavité 121 comporte une chicane 123 qui permet d'ouvrir la cavité 121 du côté de la surface inférieure 15 comme expliqué précédemment. Cette chicane 123 peut par exemple être formée par deux excroissances 1231 faisant saillie radialement de la cavité 121 et correspondant à deux ouvertures 1232 réalisées dans la surface inférieure 15, comme on peut le voir sur la figure 5. Une telle chicane 123 permet de garantir une circulation d'air à l'intérieur de la cavité 121 mais également d'empêcher l'accessoire 22 de tomber au travers de la cavité 121 au cas où ce dernier n'aurait pas été retenu par le dispositif d'arrêt 122 décrit précédemment.

Avantageusement, la zone de désinfection 12 comporte une lampe, préférentiellement une lampe UV, émettant une lumière bleue. En effet, il est connu que la lumière ayant une longueur d'ondes correspondant au bleu et/ou aux Ultra Violets a des propriétés de désinfection notables. Cela garantit ainsi une bonne hygiène de l'accessoire 22, en particulier lorsque ce dernier comprend une brosse 221 avec des poils d'une longueur conséquente. En effet, il est connu que de tels poils rendent difficile le séchage de la brosse et donc favorisent les problèmes d'hygiène de la brosse (prolifération bactérienne et/ou microbienne, moisissures, etc.).

Préférentiellement, la zone de désinfection 12 comporte un dispositif de commande spécifique, comme par exemple un micro contrôleur dédié à la gestion de l'allumage et de l'extinction de la lampe. En d'autres termes, il est possible, grâce à ce dispositif de commande spécifique de dissocier la gestion de la zone de désinfection 12 et la gestion de la zone de chargement 11 qui comporte un autre dispositif de commande. Ainsi, il n'est pas nécessaire d'alimenter en énergie la zone de chargement 11, c'est-à-dire de procéder au chargement de la batterie du corps 21 pour également alimenter en énergie la lampe et donc procéder au nettoyage de l'accessoire 22.

De manière avantageuse, ledit dispositif de commande spécifique comprend un compteur de temps permettant de décompter le temps d'allumage de la lampe et donc le temps de nettoyage de l'accessoire. Ledit dispositif de commande spécifique est alors en outre conçu pour :
- déclencher ledit compteur de temps lorsque le corps 21 commence à coopérer avec la zone de chargement 11, et pour simultanément
- allumer ladite lampe émettant une lumière bleue,
- puis, éteindre ladite lampe émettant une lumière bleue lorsque le compteur de temps atteint une valeur prédéfinie (ou valeur seuil), et ce indépendamment de l'état de chargement de la batterie du corps 21.

Préférentiellement, ladite valeur prédéfinie est définie par conception et est comprise entre 1h et 5h, avantageusement entre 2h et 4h, en étant par exemple égale à 3h. Ainsi, quelle que soit la durée de chargement de la batterie du corps 21, la durée d'exposition de l'accessoire 22 à la lampe émettant une lumière bleue, c'est-à-dire la durée de nettoyage de l'accessoire 22, sera toujours identique, ce qui garantit systématiquement un nettoyage complet de ce dernier.

Préférentiellement, la zone de désinfection 12 comporte un indicateur visuel conçu pour permettre à un utilisateur de vérifier l'allumage de la lampe. Cela permet à l'utilisateur de s'assurer de la présence de la lumière bleue à l'intérieur de la cavité 121 et donc de la bonne désinfection de l'accessoire 22 que l'utilisateur a placé dans la zone de désinfection 12. De manière astucieuse, l'indicateur visuel est formé par le chanfrein 1221. Ainsi, cela permet à l'utilisateur de visualiser un anneau de couleur bleue autour de la zone de désinfection 12, y compris lorsque l'accessoire 22 coopère avec la zone de désinfection 12. Cela confère à l'utilisateur un indicateur visuel le rassurant sur le bon fonctionnement de la base d'accueil 1 avec une solution relativement simple et bon marché.

Comme on peut le voir sur les figures 1 à 5, lesdites zone de chargement 11 et zone de désinfection 12 sont distinctes l'une de l'autre, c'est-à-dire qu'elles ne sont pas superposées, pas confondues. Par exemple, les zone de chargement 11 et zone de désinfection 12 peuvent être distantes de quelques millimètres, par exemple entre 4 mm et 10 mm préférentiellement 6.7 mm de sorte à ne pas être en contact l'une de l'autre. Cela garantit l'indépendance entre les zone de chargement 11 et de désinfection 12, ce qui permet d'améliorer l'hygiène de la base d'accueil 1 et son ergonomie.

Comme mentionné précédemment, la base d'accueil 1 peut également comprendre une zone de stockage 13. Une telle zone de stockage 13 est illustrée dans le deuxième mode de réalisation de l'invention représenté aux figures 9 à 11. La zone de stockage 13 est alors conçue pour coopérer avec l'accessoire 22 ou un autre accessoire de manière à assurer le maintien de ce dernier. En d'autres termes, la base d'accueil 1 est alors formée par trois zones distinctes : la zone de chargement 11, la zone de désinfection 12 et la zone de stockage 13. Cela permet à la base d'accueil 1 de pouvoir accueillir simultanément trois éléments de l'appareil 2 de soin de la peau: le corps 21, l'accessoire 22 et un autre accessoire 22'. En effet, il est fréquent que l'appareil 2 de soin de la peau puisse être équipé de différents accessoires 22, 22', par exemple ayant des duretés de brosse différentes, ce qui permet à l'utilisateur d'effectuer différents soins sur sa peau avec le même appareil. Grâce à ce mode de réalisation de l'invention, il devient alors possible pour l'utilisateur de disposer différents accessoires 22, 22' sur la base d'accueil 1, ce qui limite le risque de perte de ces accessoires.

Comme on peut le voir sur la figure 9, la zone de stockage 13 comprend un renfoncement 132 situé entre la surface supérieure 14 et la surface inférieure 15. Le renfoncement 132 est ouvert sur la surface supérieure 14 mais pas sur la surface inférieure 15. Ainsi le renfoncement 132 comporte un fond 133 situé entre la surface supérieure 14 et la surface inférieure 15. Ce renfoncement 132 permet d'accueillir au moins une partie de l'accessoire 22, et avantageusement les moyens d'accrochage 222.

Comme le montrent les figures 9 et 10, la zone de stockage 13 comporte un plot 131 conçu pour coopérer avec ledit accessoire 22 ou un autre accessoire de manière à ce que la brosse 221 soit stockée à l'air libre. En effet, le plot 131 constitue un obstacle qui vient entrer en collision avec la brosse 221 de l'accessoire 22 ce qui va dissuader l'utilisateur de positionner la brosse 221 au contact du plot 131, notamment par manque de stabilité. Cela va donc empêcher l'utilisateur de disposer la brosse 221 à l'intérieur de la zone de stockage 13 ce qui pourrait empêcher le séchage de la brosse 221 et donc dégrader l'hygiène de cette dernière. Le plot 131 fait sailli du fond 133 en direction de la surface supérieure 14, sans toutefois dépasser de cette dernière. Ainsi, le renfoncement 132 a sensiblement la forme d'un anneau.

Comme expliqué précédemment, l'accessoire 22 comporte un moyen d'accrochage 222 avantageusement de forme circulaire. Ainsi, le plot 131 est conçu pour accueillir autour de lui le dispositif d'accrochage 222. Cela permet d'améliorer la stabilité et le maintien de l'accessoire 22 au sein de la zone de stockage 13, y compris si la base d'accueil 1 est déplacée.

## Revendications

1. Base d'accueil (1) pour appareil (2) de soin de la peau, ledit appareil (2) comprenant un corps (21) et un accessoire (22), le corps comportant un moteur électrique relié à une batterie, le corps (21) pouvant évoluer entre une configuration de travail dans laquelle l'accessoire (22) est accroché sur le corps (21) de sorte à être mis en mouvement par le moteur électrique, et une position de repos dans laquelle l'accessoire (22) est indépendant du corps (21), la base d'accueil (1) comportant une zone de chargement (11) de l'appareil (2) de soin de la peau conçue pour coopérer avec le corps (21) de manière à recharger la batterie, une zone de désinfection (12) de l'accessoire (22) conçue pour coopérer avec l'accessoire (22) de manière à désinfecter l'accessoire (22), **caractérisée en ce que** lesdites zone de chargement (11) et zone de désinfection (12) sont distinctes l'une de l'autre et **en ce que** la base d'accueil (1) comprend un dispositif de blocage (112) conçu pour empêcher le corps (21) de coopérer avec la zone de chargement (11) si le corps (21) est en configuration de fonctionnement.

2. Base d'accueil (1) selon la revendication précédente, ladite base d'accueil (1) s'étendant entre une surface supérieure (14) et une surface inférieure (15), **caractérisée en ce que** la zone de chargement (11) comporte une chambre (111) s'étendant et ouverte depuis la surface supérieure (14) vers la surface inférieure (15).

3. Base d'accueil (1) selon la revendication précédente, **caractérisé en ce que** le dispositif de blocage (112) comporte une surface pleine située en retrait de la surface supérieure (14) de manière à fermer la chambre (111) en direction de la surface inférieure (15).

4. Base d'accueil (1) selon l'une quelconque des revendications précédentes, **caractérisée en ce que** la zone de chargement (11) comporte un dispositif de chargement sans contact, préférentiellement par induction.

5. Base d'accueil (1) selon l'une quelconque des revendications précédentes, ladite base d'accueil (1) s'étendant entre une surface supérieure (14) et une surface inférieure (15), **caractérisée en ce que** la zone de désinfection (12) comporte une cavité (121) s'étendant et ouverte depuis la surface supérieure (14) vers la surface inférieure (15).

6. Base d'accueil (1) selon la revendication précédente, **caractérisée en ce que** la cavité (121) est également ouverte du côté de la surface inférieure (15).

7. Base d'accueil (1) selon l'une quelconque des revendications 5 ou 6 **caractérisée en ce que** la zone de désinfection (12) comporte un dispositif d'arrêt (122) conçu pour coopérer avec l'accessoire (22) de manière à supporter ce dernier au sein de la cavité (121), le dispositif d'arrêt (122) comportant avantageusement un chanfrein (1221).

8. Base d'accueil (1) selon la revendication précédente **caractérisée en ce que** le dispositif d'arrêt (122) comporte des ergots d'appui (1222).

9. Base d'accueil (1) selon l'une quelconque des revendications précédentes, la zone de désinfection (12) comportant une lampe émettant une lumière bleue, **caractérisée en ce que** la zone de désinfection (12) comporte un indicateur visuel conçu pour permettre à un utilisateur de vérifier l'allumage de la lampe.

10. Base d'accueil (1) selon la revendication précédente et selon la revendication 7 **caractérisée en ce que** l'indicateur visuel est formé par le chanfrein (1221).

11. Base d'accueil (1) selon la revendication 9 ou 10 **caractérisée en ce que** la zone de désinfection (12) comporte un dispositif de commande spécifique conçu pour contrôler l'allumage et l'extinction de ladite lampe émettant une lumière bleue de manière automatique et indépendamment du contrôle de la zone de chargement (11).

12. Base d'accueil (1) selon la revendication précédente **caractérisée en ce que** ledit dispositif de commande spécifique comprend un compteur de temps conçu pour être déclenché lorsque la zone de chargement (11) et le corps (21) coopèrent et pour envoyer un signal au dispositif de commande spécifique lorsqu'une durée seuil est atteinte, le dispositif de commande spécifique étant en outre conçu pour éteindre la lampe émettant une lumière bleue lorsque la durée seuil est atteinte.

13. Base d'accueil (1) selon l'une quelconque des revendications précédentes, **caractérisée en ce qu'**elle comprend une zone de stockage (13) conçue pour coopérer avec l'accessoire (22) ou un autre accessoire (22') de manière à assurer le maintien de ce dernier.

14. Base d'accueil (1) selon la revendication précédente, ledit accessoire (22) ou un autre accessoire (22') comportant une brosse (221), **caractérisée en ce que** la zone de stockage (13) comporte un plot (131) conçu pour coopérer avec ledit accessoire (22) ou un autre accessoire (22') de manière à ce que la brosse (221) soit stockée à l'air libre.

15. Base d'accueil (1) selon la revendication précédente, ledit accessoire (22) ou un autre accessoire (22') comportant un moyen d'accrochage (222), **caractérisée en ce que** le plot (131) est conçu pour accueillir autour de lui le dispositif d'accrochage (222).

## Patentansprüche

1. Aufnahmebasis (1) für Hautpflegeeinrichtung (2), wobei die Einrichtung (2) einen Körper (21) und ein Zubehör (22) umfasst, wobei der Körper einen elektrischen Motor umfasst, der an eine Batterie angeschlossen ist, wobei der Körper (21) zwischen einer Arbeitskonfiguration, in der das Zubehör (22) derart an dem Körper (21) angebracht ist, dass es von dem elektrischen Motor in Bewegung versetzt wird, und einer Ruheposition, in der das Zubehör (22) unabhängig vom Körper (21) ist, übergehen kann, wobei die Aufnahmebasis (1) einen Ladebereich (11) der Hautpflegeeinrichtung (2) umfasst, der dazu konzipiert ist, mit dem Körper (21) derart zusammenzuwirken, dass er die Batterie auflädt, einen Desinfektionsbereich (12) für das Zubehör (22), der dazu konzipiert ist, mit dem Zubehör (22) derart zusammenzuwirken, dass er das Zubehör (22) desinfiziert, **dadurch gekennzeichnet, dass** sich der Ladebereich (11) und der Desinfektionsbereich (12) voneinander unterscheiden und dadurch, dass die Aufnahmebasis (1) eine Klemmvorrichtung (112) umfasst, die dazu konzipiert ist, den Körper (21) daran zu hindern, mit dem Ladebereich (11) zusammenzuwirken, wenn der Körper (21) in der Betriebskonfiguration ist.

2. Aufnahmebasis (1) nach dem vorstehenden Anspruch, wobei sich die Aufnahmebasis (1) zwischen einer oberen Fläche (14) und einer unteren Fläche (15) erstreckt, **dadurch gekennzeichnet, dass** der Ladebereich (11) eine Kammer (111) umfasst, die sich ausgehend von der oberen Fläche (14) zur unteren Fläche (15) erstreckt und offen ist.

3. Aufnahmebasis (1) nach dem vorstehenden Anspruch, **dadurch gekennzeichnet, dass** die Klemmvorrichtung (112) eine volle Fläche umfasst, die von der oberen Fläche (14) derart vertieft ist, dass sie die Kammer (111) in Richtung der unteren Fläche (15) verschließt.

4. Aufnahmebasis (1) nach einem der vorstehenden Ansprüche, **dadurch gekennzeichnet, dass** der Ladebereich (11) eine kontaktlose Ladevorrichtung, vorzugsweise durch Induktion, umfasst.

5. Aufnahmebasis (1) nach einem der vorstehenden Ansprüche, wobei sich die Aufnahmebasis (1) zwischen einer oberen Fläche (14) und einer unteren Fläche (15) erstreckt, **dadurch gekennzeichnet, dass** der Desinfektionsbereich (12) einen Hohlraum (121) umfasst, der sich ausgehend von der oberen Fläche (14) zur unteren Fläche (15) erstreckt und offen ist.

6. Aufnahmebasis (1) nach dem vorstehenden Anspruch, **dadurch gekennzeichnet, dass** der Hohlraum (121) auch auf der Seite der unteren Fläche (15) offen ist.

7. Aufnahmebasis (1) nach einem der Ansprüche 5 oder 6, **dadurch gekennzeichnet, dass** der Desinfektionsbereich (12) eine Arretierungsvorrichtung (122) umfasst, die dazu konzipiert ist, mit dem Zubehör (22) derart zusammenzuwirken, der dieses Letztere im Hohlraum (121) gestützt wird, wobei die Arretierungsvorrichtung (122) vorteilhafterweise eine Abschrägung (1221) umfasst.

8. Aufnahmebasis (1) nach dem vorstehenden Anspruch, **dadurch gekennzeichnet, dass** die Arretierungsvorrichtung (122) Auflagezapfen (1222) umfasst.

9. Aufnahmebasis (1) nach einem der vorstehenden Ansprüche, **dadurch gekennzeichnet, dass** der Desinfektionsbereich (12) eine Lampe umfasst, die ein blaues Licht ausgibt, **dadurch gekennzeichnet, dass** der Desinfektionsbereich (12) einen visuellen Indikator umfasst, der dazu konzipiert ist, einem Benutzer zu ermöglichen, zu überprüfen, ob die Lampe eingeschaltet ist.

10. Aufnahmebasis (1) nach dem vorstehenden Anspruch und nach Anspruch 7, **dadurch gekennzeichnet, dass** der visuelle Indikator von der Abschrägung (1221) gebildet wird.

11. Aufnahmebasis (1) nach Anspruch 9 oder 10, **dadurch gekennzeichnet, dass** der Desinfektionsbereich (12) eine besondere Steuereinheit umfasst, die dazu konzipiert ist, das Einschalten und Ausschalten der Lampe, die ein blaues Licht ausgibt, auf automatische Weise und unabhängig von der Steuerung des Ladebereichs (11) zu steuern.

12. Aufnahmebasis (1) nach dem vorstehenden Anspruch, **dadurch gekennzeichnet, dass** die besondere Steuereinheit einen Zeitzähler umfasst, der dazu konzipiert ist, ausgelöst zu werden, wenn der Ladebereich (11) und der Körper (21) zusammenwirken, um ein Signal an die besondere Steuereinheit zu senden, wenn eine Schwellendauer erreicht ist, wobei die besondere Steuereinheit weiter dazu konzipiert ist, die Lampe, die ein blaues Licht ausgibt, auszuschalten, wenn die Schwellendauer erreicht ist.

13. Aufnahmebasis (1) nach einem der vorstehenden Ansprüche, **dadurch gekennzeichnet, dass** sie einen Lagerbereich (13) umfasst, der dazu konzipiert ist, mit dem Zubehör (22) oder einem anderen Zubehör (22') derart zusammenzuwirken, dass das Halten dieses Letzteren sichergestellt wird.

14. Aufnahmebasis (1) nach dem vorstehenden Anspruch, wobei das Zubehör (22) oder ein anderes Zubehör (22') eine Bürste (221) umfasst, **dadurch gekennzeichnet, dass** der Lagerbereich (13) einen Stift (131) umfasst, der dazu konzipiert ist, mit dem Zubehör (22) oder einem anderen Zubehör (22') derart zusammenzuwirken, dass die Bürste (221) im Freien gelagert wird.

15. Aufnahmebasis (1) nach dem vorstehenden Anspruch, wobei das Zubehör (22) oder ein anderes Zubehör (22') ein Befestigungsmittel (222) umfasst, **dadurch gekennzeichnet, dass** der Stift (131) dazu konzipiert ist, die Befestigungsvorrichtung (222) um ihn herum aufzunehmen.

## Claims

1. Docking station (1) for skin care device (2), said device (2) comprising a body (21) and an accessory (22), the body comprising an electric motor connected to a battery, the body (21) being able to evolve between a work configuration wherein the accessory (22) is fastened on the body (21), so as to be moved by the electric motor, and a rest position, wherein the accessory (22) is independent from the body (21), the docking station (1) comprising a charging area (11) of the skin care device (2) designed to engage with the body (21) so as to recharge the battery, a disinfection area (12) of the accessory (22) designed to engage with the accessory (22), so as to disinfect the accessory (22), **characterized in that** said charging area (11) and disinfection area (12) are distinct from one another, and **in that** the docking station (1) comprises a blocking device (112), designed to prevent the body (21) from engaging with the charging area (11) if the body (21) is in operating configuration.

2. Docking station (1) according to the preceding claim, said docking station (1) extending between an upper surface (14) and a lower surface (15), **characterized in that** the charging area (11) comprises a chamber (111) extending and open from the upper surface (14) to the lower surface (15).

3. Docking station (1) according to the preceding claim, **characterized in that** the blocking device (112) comprises a solid surface located set back from the upper surface (14) so as to close the chamber (111) in the direction of the lower surface (15).

4. Docking station (1) according to any one of the preceding claims, **characterized in that** the charging area (11) comprises a contactless charging device, preferably by induction.

5. Docking station (1) according to any one of the preceding claims, said docking station (1) extending between an upper surface (14) and a lower surface (15), **characterized in that** the disinfection area (12) comprises a cavity (121) extending and open from the upper surface (14) to the lower surface (15).

6. Docking station (1) according to the preceding claim, **characterized in that** the cavity (121) is also open from the side of the lower surface (15).

7. Docking station (1) according to any one of claims 5 or 6, **characterized in that** the disinfection area (12) comprises a stop device (122), designed to engage with the accessory (22) so as to support the latter within the cavity (121), the stop device (122) advantageously comprising a chamfer (1221).

8. Docking station (1) according to the preceding claim, **characterized in that** the stop device (122) comprises support lugs (1222).

9. Docking station (1) according to any one of the preceding claims, the disinfection area (12) comprising a lamp emitting a blue light, **characterized in that** the disinfection area (12) comprises a visual indicator, designed to enable a user to verify the illumination of the lamp.

10. Docking station (1) according to the preceding claim and according to the claim 7, **characterized in that** the visual indicator is formed by the chamfer (1221).

11. Docking station (1) according to claim 9 or 10, **characterized in that** the disinfection area (12) comprises a specific control device, designed to control the illumination and the extinction of said lamp emitting a blue light automatically and independently from controlling the charging area (11).

12. Docking station (1) according to the preceding claim, **characterized in that** said specific control device comprises a time counter, designed to be triggered when the charging area (11) and the body (21) engage and to send a signal to the specific control device when a threshold duration is reached, the specific control device further being designed to turn off the lamp emitting a blue light when the threshold duration is reached.

13. Docking station (1) according to any one of the preceding claims, **characterized in that** it comprises a storage area (13), designed to engage with the accessory (22) or another accessory (22'), so as to ensure the holding of the latter.

14. Docking station (1) according to the preceding claim, said accessory (22) or another accessory (22') comprising a brush (221), **characterized in that** the storage area (13) comprises a pad (131), designed to engage with said accessory (22) or another accessory (22'), such that the brush (221) is stored in the open air.

15. Docking station (1) according to the preceding claim, said accessory (22) or another accessory (22') comprising a fastening means (222), **characterized in that** the pad (131) is designed to receive the fastening device (222) around it.
